# EUROPEAN PATENT APPLICATION

(11) **EP 3 338 848 A1**
(43) Date of publication of application: **27.06.2018**
(21) Application number: 17208425.3
(22) Date of filing: 19.12.2017
(51) Int. Cl.: A61M 25/00, A61B 5/042, A61B 34/20, A61B 17/00, A61B 18/00

(54) **TWO-PIECE CATHETER HANDLE**

(30) Priority: 20.12.2016 US 201662436748 P; 23.10.2017 US 201715790696
(71) Applicant: Biosense Webster (Israel) Ltd., Yokneam 2066717 (IL)
(72) Inventor: KHUDISH, Oleg, 2066717 Yokneam (IL)
(74) Representative: Carpmaels & Ransford LLP

(57) **Abstract**

Described embodiments include an apparatus that includes an intrabody catheter and a distal handle-piece connected to the intrabody catheter. The distal handle-piece includes a distal-handle-piece connector configured to reversibly electrically couple the distal handle-piece to a proximal handle-piece via a proximal-handle-piece connector of the proximal handle-piece, such that the proximal handle-piece and the distal handle-piece are held adjacent to one another. Other embodiments are also described.

## Description

### CROSS-REFERENCE TO RELATED APPLICATION

The present application claims the benefit of US Provisional Patent Application 62/436,748, entitled "Two-piece catheter handle," filed December 20, 2016, whose disclosure is incorporated herein by reference.

### FIELD OF THE INVENTION

The present invention relates generally to the field of medical devices, and particularly to intrabody catheters, such as intracardiac catheters.

### BACKGROUND

US Patent Application Publication 2013/0296729 describes a catheter having a split handle with a two piece connector that facilities reuse of a portion of the catheter assembly. Electronic circuitry that was typically placed in the operator controlled handle of the device is moved to the two-piece connector so that the electronic circuitry may be separated from the operator controlled handle for ease of reprocessing.

### SUMMARY OF THE INVENTION

There is provided, in accordance with some embodiments of the present invention, an apparatus that includes an intrabody catheter and a distal handle-piece connected to the intrabody catheter. The distal handle-piece includes a distal-handle-piece connector configured to reversibly electrically couple the distal handle-piece to a proximal handle-piece via a proximal-handle-piece connector of the proximal handle-piece, such that the proximal handle-piece and the distal handle-piece are held adjacent to one another.

In some embodiments,
the distal handle-piece further includes a distal-handle-piece body coupled to the distal-handle-piece connector,
the proximal handle-piece further includes a proximal-handle-piece body coupled to the proximal-handle-piece connector, and
the distal-handle-piece connector is configured to reversibly electrically couple the distal handle-piece to the proximal handle-piece such that less than 5 mm separate between the distal-handle-piece body and the proximal-handle-piece body.

In some embodiments, the distal-handle-piece connector is configured to reversibly electrically couple the distal handle-piece to the proximal handle-piece such that the distal-handle-piece body contacts the proximal-handle-piece body.

In some embodiments, the distal handle-piece is fixedly connected to the intrabody catheter.

In some embodiments, the distal-handle-piece connector includes a plurality of pins configured to reversibly electrically couple the distal handle-piece to the proximal handle-piece by plugging into a plurality of sockets in the proximal-handle-piece connector.

In some embodiments,
the distal-handle-piece connector is shaped to define a plurality of sockets,
the proximal-handle-piece connector includes a plurality of pins, and
the distal-handle-piece connector is configured to reversibly electrically couple the distal handle-piece to the proximal handle-piece by virtue of the sockets being configured to receive the pins.

In some embodiments, the distal-handle-piece connector includes a plurality of electrically-conductive terminals configured to reversibly electrically couple the distal handle-piece to the proximal handle-piece by mating with a plurality of complementary electrically-conductive terminals belonging to the proximal-handle-piece connector.

In some embodiments, the apparatus further includes the proximal-handle-piece connector.

In some embodiments, each of the distal-handle-piece connector and the proximal-handle-piece connector does not include a cable.

In some embodiments, the proximal handle-piece includes analog-to-digital conversion circuitry, configured to digitize analog signals received from the intrabody catheter.

In some embodiments, the distal handle-piece further includes a component selected from the group of components consisting of: overvoltage-protection circuitry, and a power-supply sequencer.

In some embodiments, the distal handle-piece includes a multiplexer, configured to selectively pass a plurality of signals, received from the intrabody catheter, to the proximal handle-piece.

There is further provided, in accordance with some embodiments of the present invention, a proximal handle-piece, including a proximal-handle-piece body and a proximal-handle-piece connector. The proximal-handle-piece connector is coupled to the proximal-handle-piece body, and is configured to reversibly electrically couple the proximal handle-piece to a distal handle-piece, which is connected to an intrabody catheter, such that the proximal handle-piece and the distal handle-piece are held adjacent to one another.

In some embodiments, the proximal-handle-piece body includes analog-to-digital conversion circuitry configured to digitize analog signals received from the intrabody catheter.

In some embodiments, the proximal-handle-piece connector includes a plurality of electrically-conductive terminals configured to reversibly electrically couple the proximal handle-piece to the distal handle-piece by mating with a plurality of complementary electrically-conductive terminals belonging to the distal handle-piece.

There is further provided, in accordance with some embodiments of the present invention, a method that includes forming a catheter handle, by coupling a proximal-handle-piece connector of a proximal handle-piece to a distal-handle-piece connector of a distal handle-piece that is coupled to an intrabody catheter, such that the proximal handle-piece and the distal handle-piece are held adjacent to one another. The method further includes, subsequently to coupling the proximal-handle-piece connector to the distal-handle-piece connector, controlling the intrabody catheter, using the catheter handle.

In some embodiments, the distal handle-piece is a first distal handle-piece and the intrabody catheter is a first intrabody catheter, and the method further includes:
decoupling the proximal handle-piece from the first distal handle-piece; and
subsequently to decoupling the proximal handle-piece from the first distal handle-piece, coupling the proximal handle-piece to a second distal handle-piece that is coupled to a second intrabody catheter.

In some embodiments, coupling the proximal handle-piece to the second distal handle-piece includes coupling the proximal handle-piece to the second distal handle-piece while the proximal handle-piece is connected to a power source.

The present invention will be more fully understood from the following detailed description of embodiments thereof, taken together with the drawing.

### BRIEF DESCRIPTION OF THE DRAWING

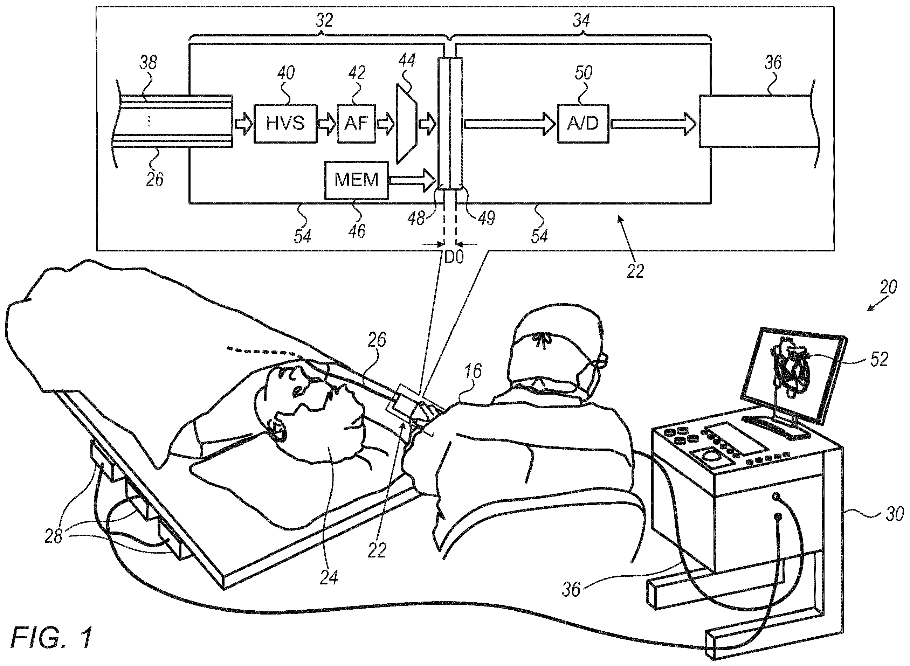

Fig. 1 is a schematic illustration of an intracardiac mapping system, in accordance with some embodiments of the present invention.

### DETAILED DESCRIPTION OF EMBODIMENTS

### OVERVIEW

For safety reasons, an intrabody catheter is typically discarded after use, due to the catheter having come into contact with the body of a subject. In many cases, the catheter is fixedly coupled to a handle, such that, when discarding the catheter, there is no choice but to also discard the handle. Unfortunately, however, catheter handles often include relatively expensive circuitry or other components.

Embodiments of the present invention therefore provide a catheter handle that is partly reusable. The handle comprises a distal handle-piece, which, typically, is fixedly coupled to the catheter, along with a proximal handle-piece that is reversibly coupled to the distal handle-piece. After the catheter is used, the proximal handle-piece is decoupled from the distal handle-piece. The catheter and the distal handle-piece are then discarded, while the proximal handle-piece is reused with the next catheter. Advantageously, relatively expensive circuitry, such as analog-to-digital (A/D) conversion circuitry, is placed in the proximal handle-piece, such that this circuitry need not be discarded.

Typically, the two pieces of the handle are coupled to one another (e.g., by a pin-and-socket connector) such that the two pieces of the handle are held adjacent to one another. One advantage of such an embodiment is that the handle-pieces are moveable together; that is, an operator may move the entire handle at once, without needing to separately grasp each of the pieces of the handle. Another advantage is that relatively little noise is introduced into the analog signals received from the distal end of the catheter before these signals are converted by the A/D conversion circuitry in the proximal handle-piece. If, on the other hand, the proximal handle-piece were remotely coupled to the distal handle-piece (e.g., via a cable), the analog signals from the distal end of the catheter would need to travel a greater distance before reaching the A/D conversion circuitry, leading to the introduction of more noise.

In some embodiments, the catheter handle described herein is used with an intracardiac mapping catheter that includes a large number of electrodes, such that a large number of signals are received from the catheter. In such embodiments, a multiplexer is typically placed in the distal piece of the handle, such that the connector is required to carry only a small number of signals (e.g., only one signal) at any given time. Thus, advantageously, the connector may be smaller, less expensive, and/or less prone to malfunction than would otherwise be the case.

### SYSTEM DESCRIPTION

Reference is made to Fig. 1, which is a schematic illustration of an intracardiac mapping system 20, in accordance with some embodiments of the present invention. System 20 comprises an intracardiac mapping catheter 26, comprising, at its distal end, a plurality of mapping electrodes. For example, the distal end of catheter 26 may comprise a basket, on which a plurality of mapping electrodes are disposed. During an intracardiac mapping procedure, a physician 16 inserts the distal end of catheter 26 into the heart of a subject 24, and contacts the tissue of the heart, at a plurality of locations, with the distal end of the catheter, such that, at each of the locations, a plurality of electrocardiographic (ECG) signals are acquired by the electrodes. Following suitable processing, as further described below, these signals are used to construct an electroanatomical map 52 of the subject's heart.

Typically, the number of electrodes in catheter 26 is relatively large; in some embodiments, for example, catheter 26 comprises more than 250 electrodes. Hence, at each location of the heart at which the distal end of the catheter is placed, the catheter may acquire a relatively large number of signals, roughly simultaneously.

In some embodiments, system 20 comprises a magnetic tracking subsystem, which is used to ascertain the position and orientation of catheter 26 during the procedure. Such a magnetic tracking subsystem typically comprises one or more magnetic-field generators 28, which generate magnetic fields in the vicinity of subject 24. These generated magnetic fields induce voltages in coils disposed at the distal end of catheter 26. Based on these induced voltages, the position and orientation of the catheter are ascertained. This information is then associated with the electrocardiographic information acquired by the electrodes, such that electroanatomical map 52 may be constructed.

Alternatively or additionally to a magnetic tracking subsystem, an impedance-based tracking subsystem may be used, as described, for example, in U.S. Patent Nos. 7,756,576 and 7,536,218, the respective disclosures of which are incorporated herein by reference.

During the procedure, a processor located in a console 30 receives, via a cable 36, the above-mentioned tracking and electrocardiographic signals, typically after these signals are processed as described below. Based on these signals, the processor constructs electroanatomical map 52. This map may then be displayed, stored, and/or processed in any suitable manner.

System 20 further comprises a catheter handle 22, comprising a distal handle-piece 32, which is connectable to catheter 26 (e.g., to the proximal end of catheter 26), and a proximal handle-piece 34. Distal handle-piece 32 comprises a distal-handle-piece connector 48, while proximal handle-piece 34 comprises a complementary proximal-handle-piece connector 49. Distal-handle-piece connector 48 and proximal-handle-piece connector 49 (which may be collectively referred to as a single connector) are configured to reversibly electrically couple the two handle-pieces to one another, i.e., reversibly couple the two handle-pieces to one another such that electric signals may pass from either one of the handle-pieces to the other one of the handle-pieces. To form catheter handle 22, physician 16 (or any other operator) couples the proximal-handle-piece connector to the distal-handle-piece connector. Subsequently to the formation of the catheter handle, the physician uses the catheter handle to control the catheter, e.g., by manipulating any controls (such as buttons, switches, or dials) located on one or both of the handle-pieces.

Distal-handle-piece connector 48 and proximal-handle-piece connector 49 may be of any suitable type. For example, one of the handle-piece connectors may be shaped to define one or more sockets, with the other handle-piece connector comprising one or more complementary pins configured to plug into the sockets, such that the sockets receive the pins. Alternatively, each of the connectors may comprise electrically-conductive terminals, or pads, configured to mate with complementary terminals, or pads, on the other one of the connectors.

Each of the connectors may have any suitable shape. For example, each of the connectors may be conically-shaped, or otherwise shaped to define a three-dimensional mating surface, as described, for example, in US Patent Application Publication 2017/0172652, whose disclosure is incorporated herein by reference.

Typically, the proximal handle-piece and distal handle-piece are coupled together such that they are held adjacent to one another. For example, the connectors may hold the two handle-pieces together such that the separation D0 between the respective handle-piece bodies 54 of the handle-pieces is less than 5 mm. (The "body" of a handle-piece is the portion of the handle-piece that does not include the connector, which is coupled to the connector.) For example, the connectors may hold the two handle-piece bodies in contact with one another. Typically, neither one of the connectors comprises a cable, which would cause the two handle-pieces to be coupled more remotely from one another.

As described above in the Overview, distal handle-piece 32 is typically fixedly connected to the catheter, and is therefore discarded after use, whereas proximal handle-piece 34 is typically reused with multiple catheters.

As illustrated in Fig. 1, a plurality of wires 38 run through the lumen of catheter 26. These wires carry electrocardiographic signals from the catheter (e.g., from electrodes at the distal end of the catheter) to distal handle-piece 32. In some embodiments, distal handle-piece 32 comprises a high-voltage suppressor (HVS) 40, which suppresses any high voltages in these signals, and/or an analog filter (AF) 42, which filters out noise from these signals. Alternatively or additionally, distal handle-piece 32 may comprise a memory (MEM) 46, which stores relevant data for constructing map 52. For example, memory 46 may store data related to the configuration of the electrodes at the distal end of the catheter, and/or data related to the calibration of the catheter.

Typically, wires 38 also carry tracking signals, such as the induced voltages described above, which indicate the position and orientation of the catheter.

Typically, distal handle-piece 32 comprises a multiplexer 44, which selectively passes the plurality of signals, received via wires 38, to proximal handle-piece 34. For example, multiplexer 44 may sequentially pass the signals, i.e., pass the signals one at a time, to the proximal handle-piece. The multiplexer thus facilitates using a large number of distal-end electrodes, without requiring that the handle-piece connector be capable of simultaneously passing a large number of signals. (Multiplexer 44 may alternatively be referred to as an analog switch.)

Typically, proximal handle-piece 34 comprises an analog-to-digital (A/D) converter 50, which digitizes any analog signals, such as the above-described ECG and tracking signals, received from the catheter via the distal handle-piece. Alternatively or additionally, the proximal handle-piece may comprise other circuitry, which processes these signals. The processed signals are passed to cable 36, which, as described above, delivers these signals to console 30.

As described above, an advantage of embodiments of the present invention is that the circuitry contained in the proximal handle-piece need not be discarded after use.

It is noted that the configuration of handle 22 shown in Fig. 1 is presented by way of example only, and that the scope of the present disclosure includes any suitable configuration. For example, each of the handle-pieces may include any suitable types of, and arrangement of, components.

Advantageously, some embodiments of the present invention allow the proximal handle-piece to be decoupled from the distal handle-piece (and catheter), and subsequently recoupled to the distal handle-piece or coupled to a different distal handle-piece (and catheter), while the proximal handle-piece is connected to a power source, such as a power source in console 30. Such a feature may be helpful, for example, in the event that the catheter needs to be quickly disconnected from the power source, e.g., due to activation of a defibrillator, or due to malfunction of the catheter. In some embodiments, the distal handle-piece comprises overvoltage-protection circuitry, and/or a power-supply sequencer, to help protect the distal handle-piece from overly high voltages during connection to, or disconnection from, the proximal handle-piece, while the latter is connected to the power source.

Although the present application relates mainly to electrocardiographic applications, it is noted that the embodiments described herein may be used for any suitable application in which an intrabody catheter is used, as well as other applications that do not involve an intrabody catheter. For example, the two-part handle described herein may be used for receiving ECG signals from body-surface electrodes, in that the distal handle-piece may receive the ECG signals via a cable or tube, and then pass the signals (e.g., sequentially) to the proximal handle-piece.

It will be appreciated by persons skilled in the art that the present invention is not limited to what has been particularly shown and described hereinabove. Rather, the scope of embodiments of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. Apparatus, comprising:
an intrabody catheter; and
a distal handle-piece connected to the intrabody catheter, and comprising a distal handle-piece connector integrated into the distal handle-piece, the distal handle-piece connector configured to reversibly electrically couple the distal handle-piece to a proximal handle-piece via a proximal-handle-piece connector integrated into the proximal handle-piece such that the proximal handle-piece and the distal handle-piece are held immediately adjacent to one another.

2. The apparatus according to claim 1,
wherein the distal handle-piece further comprises a distal-handle-piece body coupled to the distal-handle-piece connector,
wherein the proximal handle-piece further includes a proximal-handle-piece body coupled to the proximal-handle-piece connector, and
wherein the distal-handle-piece connector is configured to reversibly electrically couple the distal handle-piece to the proximal handle-piece such that less than 5 mm separate between the distal-handle-piece body and the proximal-handle-piece body, preferably such that the distal-handle-piece body contacts the proximal-handle-piece body.

3. The apparatus according to claim 1, wherein the distal handle-piece is fixedly connected to the intrabody catheter.

4. The apparatus according to claim 1, wherein the distal-handle-piece connector comprises a plurality of pins configured to reversibly electrically couple the distal handle-piece to the proximal handle-piece by plugging into a plurality of sockets in the proximal-handle-piece connector.

5. The apparatus according to claim 1,
wherein the distal-handle-piece connector is shaped to define a plurality of sockets, and
wherein the proximal-handle-piece connector includes a plurality of pins,
the distal-handle-piece connector being configured to reversibly electrically couple the distal handle-piece to the proximal handle-piece by virtue of the sockets being configured to receive the pins.

6. The apparatus according to claim 1, wherein the distal-handle-piece connector comprises a plurality of electrically-conductive terminals configured to reversibly electrically couple the distal handle-piece to the proximal handle-piece by mating with a plurality of complementary electrically-conductive terminals belonging to the proximal-handle-piece connector.

7. The apparatus according to claim 1, further comprising the proximal-handle-piece connector.

8. The apparatus according to claim 7, wherein each of the distal-handle-piece connector and the proximal-handle-piece connector does not comprise a cable, or wherein the proximal handle-piece comprises analog-to-digital conversion circuitry, configured to digitize analog signals received from the intrabody catheter.

9. The apparatus according to claim 1, wherein the distal handle-piece further comprises, (i) a component selected from the group of components consisting of: overvoltage-protection circuitry, and a power-supply sequencer, or (ii) a multiplexer, configured to selectively pass a plurality of signals, received from the intrabody catheter, to the proximal handle-piece.

10. A proximal handle-piece, comprising:
a proximal-handle-piece body; and
a proximal-handle-piece connector, integrated into the proximal-handle-piece body, the proximal-handle-piece connector configured to reversibly electrically couple the proximal handle-piece to a distal handle-piece, which is connected to an intrabody catheter, such that the proximal handle-piece and the distal handle-piece are immediately held adjacent to one another.

11. The proximal handle-piece according to claim 10, wherein the proximal-handle-piece body comprises analog-to-digital conversion circuitry configured to digitize analog signals received from the intrabody catheter.

12. The proximal handle-piece according to claim 10, wherein the proximal-handle-piece connector comprises a plurality of electrically-conductive terminals configured to reversibly electrically couple the proximal handle-piece to the distal handle-piece by mating with a plurality of complementary electrically-conductive terminals belonging to the distal handle-piece.

13. A method, comprising:
forming a catheter handle, by directly coupling a proximal-handle-piece connector of a proximal handle-piece to a distal-handle-piece connector of a distal handle-piece that is coupled to an intrabody catheter, such that the proximal handle-piece and the distal handle-piece are held immediately adjacent to one another; and
subsequently to coupling the proximal-handle-piece connector to the distal-handle-piece connector, controlling the intrabody catheter, using the catheter handle.

14. The method according to claim 13,
wherein the proximal handle-piece includes a proximal-handle-piece body, which is coupled to the proximal-handle-piece connector,
wherein the distal handle-piece includes a distal-handle-piece body, which is coupled to the distal-handle-piece connector, and
wherein coupling the proximal-handle-piece connector to the distal-handle-piece connector comprises coupling the proximal-handle-piece connector to the distal-handle-piece connector such that less than 5 mm separate between the distal-handle-piece body and the proximal-handle-piece body, preferably such that the distal-handle-piece body contacts the proximal-handle-piece body.

15. The method according to claim 13, wherein the distal handle-piece is a first distal handle-piece and the intrabody catheter is a first intrabody catheter, and wherein the method further comprises:
decoupling the proximal handle-piece from the first distal handle-piece; and
subsequently to decoupling the proximal handle-piece from the first distal handle-piece, coupling the proximal handle-piece to a second distal handle-piece that is coupled to a second intrabody catheter, preferably wherein coupling the proximal handle-piece to the second distal handle-piece comprises coupling the proximal handle-piece to the second distal handle-piece while the proximal handle-piece is connected to a power source.
